Europäisches Patentamt
European Patent Office
Office européen des brevets

(11) Veröffentlichungsnummer: **0 305 434 B1**

(12) # EUROPÄISCHE PATENTSCHRIFT

(45) Veröffentlichungstag der Patentschrift: **07.06.95**

(51) Int. Cl.6: **C12P 7/06**, C12P 7/16, C12P 7/26

(21) Anmeldenummer: **88902076.4**

(22) Anmeldetag: **10.03.88**

(86) Internationale Anmeldenummer: **PCT/AT88/00012**

(87) Internationale Veröffentlichungsnummer: **WO 88/07090 (22.09.88 88/21)**

Verbunden mit 88890048.7/0282474 (europäische Anmeldenummer/Veröffentlichungsnummer) durch Entscheidung vom 25.10.93.

(54) **VERFAHREN ZUR KONTINUIERLICHEN VERGÄRUNG VON KOHLENHYDRATHÄLTIGEN MEDIEN MIT HILFE VON BAKTERIEN.**

(30) Priorität: **10.03.87 AT 566/87**

(43) Veröffentlichungstag der Anmeldung: **08.03.89 Patentblatt 89/10**

(45) Bekanntmachung des Hinweises auf die Patenterteilung: **07.06.95 Patentblatt 95/23**

(84) Benannte Vertragsstaaten: **AT BE CH DE ES FR GB GR IT LI LU NL SE**

(56) Entgegenhaltungen:
**EP-A- 0 112 459**
**GB-A- 2 054 645**
**GB-A- 2 113 711**

(73) Patentinhaber: **Effenberger, Helmut, Dipl.-Ing. Dr.**
**Ottakringerstrasse 49/2/15**
**A-1160 Wien (AT)**

(72) Erfinder: **SCHMIDT, Alfred**
**Pacassistrasse 29**
**A-1130 Wien (AT)**
Erfinder: **STEINER, Eva**
**Kleinengersdorferstr. 8**
**A-2100 Korneuburg (AT)**
Erfinder: **SALZBRUNN, Wolfgang**
**Bräunlichgasse 11**
**A-2700 Wr. Neustadt (AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Biotechnology & Bioengineering, vol. 26, no. 8, August 1984, John Wiley & Sons, Inc. (New York, US) M. Nagashima et al. pp. 992-997**

**Chemical Abstracts, vol. 100, no. 25, 18 June 1984 (Columbus, Ohio, US) s. p. 443, abstract no. 207895z, & JP,A,5945891 (MITSUBISHI KAKOKI KAISHA LTD.) 14 March 1984**

**Biotechnology & Bioengineering Symposium, No. 17, 1986, John Wiley & Sons, Inc. (New York, US) C. Sola et al. pp. 519-533**

**Chemical Abstracts, vol. 102, no. 13, 01 April 1985 (Columbus, Ohio, US) W.J. Groot et al. s. p. 544, abstract no. 111418m, & Biotechnol. Lett. 1984, 6(12), 789-92**

(74) Vertreter: **Sonn, Helmut et al**
**Patentanwälte**
**Sonn, Pawloy, Weinzinger & Wolfram**
**Riemergasse 14**
**A-1010 Wien (AT)**

## Beschreibung

Die Erfindung bezieht sich auf ein Verfahren zur Vergärung von kohlenhydrathältigen Medien mit Hilfe von Bakterien, welche Butanol, Aceton, Ethanol und/oder Isopropanol bilden, welches Verfahren wenigstens zweistufig ausgeführt wird, wobei im ersten Verfahrensabschnitt im wesentlichen die Bakterien kontinuierlich kultiviert und im zweiten, kontinuierlich oder chargenweise geführten Verfahrensabschnitt im wesentlichen das Produkt gebildet wird.

Gemäß Biotechnology and Bioengineering, Band 26 (8), werden die verwendeten Zellen von Saccharomyces cerevisiae in einem Trägermaterial immobilisiert, wobei das Trägermaterial aus verschiedenen Polymeren oder Hydrogelen bestehen kann. Die separate Herstellung und damit auch die Immobilisierung der Bakterien erfolgt gemäß dieser Schrift kontinuierlich außerhalb und vor Beschickung des Reaktors (oder der Reaktoren) mit dem Zellmaterial.

CA 100:207895z, 1984 betrifft ein Verfahren zur chargenweisen Ethanolherstellung, wobei Nährmedium mit Saccharomyces formosensis Nakazawa inokuliert, die Mischung sodann beispielsweise mit 52 % Polyvinylacetatkügelchen versetzt und in einem versiegelten Fermentor mit einmaligem Austausch des Nährmediums zweimal inkubiert wurde. Von einer kontinuierlichen Kultivierung der (immobilisierten) Bakterien im ersten Verfahrensabschnitt kann nicht die Rede sein, auch nicht von einer kontinuierlichen Führung des zweiten Verfahrensabschnittes. Auch werden durch dieses Verfahren nicht die mit der Herstellung von Butanol in Verbindung stehenden Probleme (z.B. die Toxizität des Butanols ab etwa 0,03 %) gelöst.

In einem Artikel von C. Solà et al. in Biotechnology & Bioengineering Symp. No. 17, New York (1986), wurde vorgeschlagen, Hefezellen in Karrageengel-Kügelchen zu immobilisieren und zur kontinuierlichen Ethanolherstellung in einem Festbettfermentor zu verwenden.

Gemäß CA 102: 111418m werden immobilisierte Clostridium beyerinckii-Zellen zur kontinuierlichen Fermentation von Glucose zur Butanol- bzw. Isopropanolherstellung verwendet, wobei die Produkte kontinuierlich mittels Pervaporation abgetrennt werden.

Die GB-A-2 113 711 wiederum beschreibt ein Verfahren zur Ethanolherstellung mittels kontinuierlicher Fermentation unter Verwendung eines Fließbett- bzw. Wirbelschichtfermentors, wobei die verwendeten Bakterien auf einen Träger abgeschieden werden.

Bei einem weiteren bekannten Verfahren der eingangs angegebenen Art sind zwei herkömmliche Züchtungsstufen vorgesehen, wobei man mit Hilfe der Durchflußrate bzw. mit Hilfe der Phosphatlimitierung den Verfahrensablauf so einstellt, daß in der ersten Stufe (Wachstumsstufe) die Bakterienkonzentration konstant hoch bleibt, d.h., daß sich ein Fließgleichgewicht (steady state) einstellt und daß bereits ein Teil des Substrats zu Butanol, Aceton und/oder Ethanol umgesetzt wird. In der zweiten Stufe soll dann eine möglichst hohe Ausbeute an den gewünschten Gärungsprodukten in möglichst kurzer Zeit erzielt werden. Wählt man in der ersten Stufe eine zu hohe Durchflußrate, so werden mehr Zellen ausgetragen als neue gebildet. Bei zu geringer Durchflußrate hingegen kann das Zellwachstum gegebenenfalls eingestellt werden.

Ein solches Verfahren hat den Nachteil, daß einerseits die erzielten Produktivitäten noch nicht befriedigend sind, und daß andererseits eine Langzeitstabilität der kontinuierlichen Kultur nicht gegeben ist, d.h. daß über einen längeren Zeitraum von hundert Stunden und mehr keine konstanten Produktivitäten erzielt werden können. Dies liegt darin, daß die gebildeten Produkte, nämlich Butanol, Aceton und/oder Ethanol bzw. Isopropanol für die Gärungsorganismen toxisch wirken, so daß bei einer gewissen Konzentration der gebildeten Produkte entweder die Produktbildung eingestellt oder aber die Bakterienkulturen erheblich geschädigt werden. Ein weiterer Nachteil des bekannten Verfahrens ist auch darin zu erblicken, daß halbwegs befriedigende Resultate nur mit Hexosen erzielt sind.

Der Erfindung liegt die Aufgabe zugrunde, ein Verfahren der eingangs erwähnten Art zu schaffen, mit welchem über einen langen Zeitraum hindurch konstant günstige Produktivitätswerte erzielbar sind. Außerdem soll auf Grund des Verfahrens erzielt werden, daß auch Ausgangsmaterialien eingesetzt werden können, die nicht nur Hexosen beinhalten.

Erfindungsgemäß wird diese Aufgabe dadurch gelöst, daß die Bakterien im zweiten Verfahrensschritt adhäsiv bzw. adsorptiv an offenporiges Sinterglas, Bimsstein oder ein gleichwertiges Trägermaterial immobilisiert werden, welches einen hohen $SiO_2$-Gehalt, eine offenporige Struktur, eine Porengröße von 20 - 200 $\mu$m und eine Wasseraufnahmefähigkeit von wenigstens 0,35 ml/cm$^3$ aufweist, und wobei das Verhältnis des Volumens des Trägermaterials zum Gesamtarbeitsvolumen 1:10 bis 1:1,1, beträgt.

Bei einstufigen Verfahren ist es bereits bekannt, Bakterien zu immobilisieren, u.zw. werden bei diesem bekannten Verfahren Zellen in Ca-Alginat immobilisiert, wobei Glucose als Substrat eingesetzt wird. Bei diesen bekannten Verfahren konnten die Produktivitäten zwar gesteigert werden, jedoch konnten auch hier über eine längere Gärungszeit, z.B. von mehreren Wochen, nur geringe Produktivitäten aufrechterhalten werden.

Das erfindungsgemäße Verfahren ist hinsichtlich der Durchflußrate sehr flexibel, da die lebensfähigen Bakterien in der zweiten Stufe durch Adhäsion am Trägermaterial festhaften, wobei die Durchflußrate im ersten Verfahrensabschnitt so auf den Verfahrensablauf abgestimmt werden kann, daß in diesem ersten Verfahrensabschnitt nahezu ausschließlich Bakterien gebildet werden, welche dann in die zweite Verfahrensstufe teilweise mitgenommen werden. In dieser zweiten Verfahrensstufe erhält man dann eine hohe Bakterienpopulation, da sich die lebensfähigen Bakterien an das Trägermaterial anheften, wogegen bereits abgestorbene Bakterienzellen mit dem produkt-angereicherten Medium ausgeschwemmt werden. Es kann also mit einer so hohen Durchflußrate in der ersten Stufe gearbeitet werden, daß die Konzentration an gebildeten Produkten nicht die Toxizitätsgrenze überschreitet, so daß die Bakterienkultur im wesentlichen nicht geschädigt wird. Vorteilhafterweise kann als Trägermaterial ein Material mit einer Porengröße von 50 - 100 $\mu$m eingesetzt werden, wobei das Verhältnis des Volumens des Trägermaterials zum Gesamtarbeitsvolumen 1:5 bis 1:1,6 beträgt, wodurch eine besonders wirksame Vergärung erzielt wird, insbesondere wenn in dem ersten Verfahrensabschnitt unter Durchmischung des Reaktorinhalts eine Verdünnungsrate von 0,08 bis 0,45 x $h^{-1}$, insbesondere 0,1 - 0,38 $h^{-1}$, ein pH von 4,0 - 5,5, insbesondere 4,3 bis 5,1, vorzugsweise 4,5 - 5 und/oder eine Temperatur von 30° - 40°C, insbesondere 33° - 38°C, eingehalten werden, wobei im zweiten Verfahrensabschnitt eine Verdünnungsrate von 0,14 - 0,4 x $h^{-1}$, vorzugsweise 4,2 - 4,5, und/oder eine Temperatur von 27° - 40°C, insbesondere 30° - 37°C, vorzugsweise 32° - 35°C, eingehalten werden.

Bei einer weiteren vorteilhaften Ausgestaltung des erfindungsgemäßen Verfahrens kann aus der Produktbildungsstufe Medium abgezogen, das gebildete Produkt extraktiv, vorzugsweise in an sich bekannter Weise mittels höherer Alkohole oder höherer Fettsäuren, abgetrennt und die dadurch anfallende produktarme Phase in die Produktbildungsstufe und/oder den ersten Verfahrensabschnitt rückgeführt werden. Dadurch wird erreicht, daß in zweiten Verfahrensabschnitt die Produktkonzentration niedrig gehalten wird, wodurch eine Schädigung der Bakterien hintangehalten wird, so daß immer eine sehr hohe Produktbildungsrate aufrechterhalten wird, u.zw. auch über einen sehr langen Zeitraum. Weiters wird ermöglicht, den Abtrennungsschritt auszuführen, ohne damit die eigentliche Gärstufe zu beeinträchtigen. Es kann jedoch im zweiten Verfahrensabschnitt Produkt, vorzugsweise kontinuierlich, pervaporativ aus dem Medium abgeführt werden. Dabei kann für die pervaporative Produktentnahme, eine Pervaporationsmembran, insbesonders aus Silikonkautschuk, eingesetzt werden, wobei eine Temperatur von 30° - 90°C, vorzugsweise 40° - 80°C, eingehalten wird und wobei die Dampfdruckerniedrigung pervoporationsseitig durch ein Trägergas, wie z.B. $N_2$, $H_2$, $CO_2$ oder Mischungen dieser Gase oder auch ein Fermentationsgas bzw. durch Anlegen eines Vakuums erzielt wird. Es kann jedoch im zweiten Verfahrensabschnitt eine Produktabtrennung, vorzugsweise kontinuierlich, auch mittels Gasstrippen durchgeführt werden. Dabei kann für die kontinuierliche Produktabtrennung durch Gasstrippen als Strippgas z.B. $N_2$, $H_2$, $CO_2$ oder Mischung dieser Gase bzw. Fermentationsgas eingesetzt werden, wobei das Verhältnis der Volumenströme von Flüssigkeit zu Gas 0,05:1 bis 1,6:1 und die Temperatur 30° - 90°C, vorzugsweise 40° - 80°C, betragen.

All die angeführten Verfahren zur Produktabtrennung bewirken neben der Einsetzbarkeit von unkonventionellen Zuckern eine höhere Produktivität, die Möglichkeit eines Einsatzes von höher konzentrierten Zuckerlösungen und eine hohe Langzeitstabilität.

Für eine besonders genaue Regelung des Verfahrensablaufes kann der Zulauf an Nährlösung und/oder Zuckerlösung in Abhängigkeit vom Produktgehalt des zweiten Verfahrensabschnittes geregelt werden. Dadurch lassen sich besonders genau jene Konzentrationen an Nährlösung bzw. Zucker einstellen, die für einen optimalen Verfahrensablauf erforderlich sind.

Bei einer erfindungsgemäßen Vorrichtung zur Durchführung des erfindungsgemäßen Verfahrens kann ein Bioraktor, vorzugsweise ein Rührkesselbioreaktor, und ein mit Bakterien rückhaltendem Material bestückter Reaktor, vorzugsweise ein Festbettreaktor, hintereinandergeschaltet sein, wobei an letzterem Reaktor eine externe Schlaufe zur, vorzugsweise kontinuierlichen, Produktabtrennung angeschlossen ist. Durch eine solche Reaktorkombination läßt sich auf besonders einfache Weise einerseits die Wachstumsgeschwindigkeit der eingesetzten Bakterien und andererseits die Produktbildung in der zweiten Verfahrensstufe regeln.

Dabei können innerhalb des Festbettreaktors ein oder mehrere Leiteinrichtungen zur Erzielung einer Zirkulation innerhalb des Reaktors vorgesehen sein. Dadurch wird erreicht, daß innerhalb des Reaktors an allen Stellen etwa gleiche Konzentrationen an Nährsubstrat und auch an Produkt vorherrschen. Weiters können zur Erzielung der Zirkulation örtlich Gaseinbringungseinrichtungen oder Leitungen zum gerichteten Einströmen von, vorzugsweise rückgeführtem Medium, vorgesehen sein. Im Falle von Gaseinbringung wird dabei die Zirkulation durch eine Mammutpumpenwirkung erreicht, wogegen bei gerichtetem Einströmen von Flüssigkeit die Zirkulation durch die entsprechende Flüssigkeitsgeschwindigkeit erreicht wird.

In der externen Schlaufe kann zur Produktabtrennung ein Pervaporationsmodul angeordnet sein. Bevorzugt kann in dem Pervaporationsmodul eine Membran, insbesondere eine Silikonkautschukmembran, vorgesehen sein. Dadurch wird eine besonders günstige Abtrennung des Produktes aus dem Medium erzielt, wobei eventuell durch die Anströmung der Membran im Querstrom ein Ablagern von Bakterien an der Membran verhindert wird. Schließlich kann in der externen Schlaufe zur Produktabtrennung eine Gegenstromstrippkolonne angeordnet sein. Es hängt dabei vom eingesetzten Ausgangsmaterial, von den eingesetzten Organismen und auch vom gebildeten Produkt ab, welche der Möglichkeiten zur Produktabtrennung herangezogen werden.

Zusammenfassend ist zu bemerken, daß in der ersten Verfahrensstufe eine derartig hohe Durchflußrate gewählt wird, daß sich die Bakterien in der Wachstumsphase und Säurebildungsphase befinden, ohne daß eine nennenswerte Produktbildung stattfindet. Die Bakterien entwickeln in dieser Wachstumsphase gute Adhäsionseigenschaften, die bei ihrem Übergang in den zweiten Verfahrensabschnitt dann zur Immobilisierung der Bakterien am Trägermaterial führen. Wird im ersten Verfahrensabschnitt eine zu geringe Durchflußrate gewählt, so wird das Zellwachstum, gegebenenfalls unter gleichzeitiger Produktbildung, eingestellt, und damit gehen auch die guten Adsorptionseigenschaften der Bakterien gegenüber dem Trägermaterial verloren.

Anstelle einer Immobilisierung könnten im zweiten Verfahrensabschnitt die Zellen mittels Mikrofiltration, vorzugsweise cross-flow Mikrofiltration aus dem Medium abgetrennt und rückgeführt werden, wodurch die gleiche Wirkung wie durch Immobilisierung erzielt wird.

Zusätzlich führt eine hohe Produktkonzentration in der ersten Stufe (bei niedriger Durchflußrate) zu einer Degenerierung der Bakterien, was zu einem Wechsel von der Produktbildung zur erneuten Säurenbildung führt, welcher Wechsel erfahrungsgemäß nicht mehr rückgängig gemacht werden kann. Bei zu hoher Durchflußrate in der ersten Stufe kommt es anderseits zu einem Auswaschen der Bakterien, so daß nach einiger Zeit nicht mehr genügend Bakterien für den zweiten Verfahrensabschnitt nachgeliefert werden.

Es ist also die Durchflußrate entsprechend den jeweiligen Gegebenheiten einzustellen.

In der Zeichnung sind schematisch verschiedene Ausführungsbeispiele der erfindungsgemäßen Vorrichtung dargestellt. Fig. 1 zeigt das Verfahrensschema, mit welchem das gebildete Produkt durch Pervaporation abgeschieden wird. Fig. 2 zeigt das Verfahrensschema, bei welchem die Produktabtrennung durch Extraktion erfolgt. Fig. 3 gibt das Verfahrensschema wieder, bei welchem das Produkt durch Strippen aus dem Medium ausgetrieben wird.

Bei allen drei Ausführungsbeispielen sind die gleichen Teile mit gleichen Bezugszeichen versehen. Mit 1 ist ein Vorratsgefäß bezeichnet, von welchem über eine Leitung 2 mittels einer Pumpe 3 die Nährlösung in den ersten Verfahrensabschnitt, welcher durch einen Rührkesselbioreaktor 4 gebildet ist, eingebracht wird. Von dem Rührkesselbioreaktor wird das mit Biomasse angereicherte Nährsubstrat über eine Leitung 5 und eine Pumpe 6 der zweiten Verfahrensstufe zugeführt, welche durch einen Festbettreaktor 7 gebildet ist. Von diesem Festbettreaktor geht eine Leitung 8 aus, über welche mittels einer Pumpe 9 das an Produkt angereicherte Medium aus dem Festbettreaktor abgeführt wird.

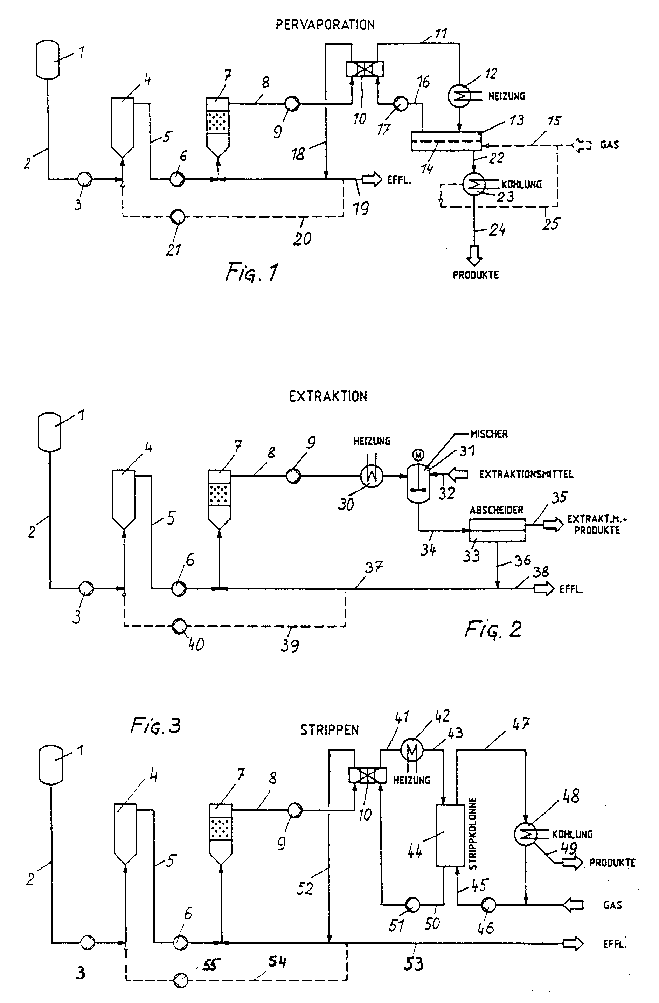

Beim Ausführungsbeispiel gemäß Fig. 1 wird das an Produkt angereicherte Medium über einen Wärmetauscher 10 geführt, in welchem es gegen rückströmendes, vom Produkt bereits befreiten Medium aufgewärmt wird. Das aufgewärmte, an Produkt reiche Medium wird über die Leitung 11 einer Heizung 12 zugeführt und von dort einem Pervaporationsmodul 13 zugeleitet. In diesem Pervaporationsmodul 13 befindet sich eine Membran 14, an deren einer Seite Medium und an deren anderer Seite ein Trägergas geführt wird. An dieser Membran wird ein Produktaustausch vom Medium zum Gas vorgenommen, wobei das Gas über eine Leitung 15 in das Pervaporationsmodul 13 eingebracht wird. Das vom Produkt befreite Medium wird über die Leitung 16 und eine Pumpe 17 dem Wärmetauscher 10 zugeführt und über die Leitung 18 wieder in den Festbettreaktor rückgeführt. Überschüssiges vom Produkt weitgehend befreites Medium wird über die Leitung 19 abgeführt. Ein Teil des Mediums wird, wie in Fig. 1 strichliert eingezeichnet, über eine Leitung 20 und eine Pumpe 21 dem Rührkesselbioreaktor zugeführt. Das an Produkt angereicherte Gas wird aus dem Pervorationsmudul 13 über die Leitung 22 einer Kühleinrichtung 23 zugeführt, in welcher das gebildete Produkt kondensiert und über die Leitung 24 abgezogen wird. Das vom Produkt befreite Gas wird über die Leitung 25 wieder dem Pervaporationsmodul 13 rückgeführt.

Beim Ausführungsbeispiel gemäß Fig. 2 wird das über die Leitung 8 und die Pumpe 9 aus dem Festbettreaktor abgezogene mit Produkt angereicherte Medium einer Heizeinrichtung 30 zugeführt, von welcher es in einen Mischer 31 gelangt. In diesen Mischer wird über die Leitung 32 Extraktionsmittel eingeleitet und das Gemisch aus Extraktionsmittel und an Produkt angereichertem Medium einem Abscheider 33 über die Leitung 34 zugeleitet. Im Abscheider 33 werden dann das mit Produkten beladene Extraktionsmittel und das vom Produkt befreite Medium voneinander getrennt, wobei das mit Produkten angereicherte Extraktionsmittel über die Leitung 35 und das vom Produkt befreite Medium über die Leitung 36 abgezogen wird. Das vom Produkt befreite Medium wird dann über die Leitung 37 in den Festbettreaktor rückgeführt, überschüssiges Medium wird über die Leitung 38 abgezogen. Wie in Fig.2 strichliert angedeutet, kann auch vom Produkt befreites Medium über eine Leitung 39 und eine Pumpe 40 in den ersten Verfahrensabschnitt rückgeführt werden. Die Trennung zwischen Extraktionsmittel und Produkt kann in herkömmlicher Weise durch Destillation u.dgl. vorgenommen werden.

Beim Ausführungsbeispiel gemäß Fig. 3 wird das über die Leitung 8 und die Pumpe 9 abgezogene, an Produkt angereicherte Medium wieder einem Wärmetauscher 10 zugeführt, von welchem es über die Leitung 41 einer Heizung 42 zugeführt wird, von wo es über die Leitung 43 in eine Strippkolonne 44 gelangt. In dieser Strippkolonne wird das an Produkt angereicherte Medium im Gegenstrom mit Gas behandelt, welches über die Leitung 45 und die Pumpe 46 in die Kolonne eingeblasen wird. Das an Produkt angereicherte Strippgas wird über die Leitung 47 einer Kühleinrichtung 48 zugeführt, in welcher die Produkte kondensieren und über die Leitung 49 abgezogen werden. Das vom Produkt freie Gas wird wieder über die Pumpe 46 und die Leitung 47 in die Strippkolonne 44 rückgeführt. Das vom Produkt befreite Medium wird über die Leitung 50 und die Pumpe 51 dem Wärmetauscher 10 zugeführt, in welchem das noch warme Medium gegen frisches, vom Festbettreaktor 7 kommenden Medium geführt wird. Vom Wärmetauscher 10 gelangt das vom Produkt befreite Medium über die Leitung 52 in den Festbettreaktor 7. Auch in vorliegendem Fall wird überschüssiges Medium über eine Leitung 53 aus dem System abgezogen. Die strichliert eingezeichnete Leitung 54 und die Pumpe 55 dienen wieder zur Rückführung von vom Produkt befreiten Medium in den ersten Verfahrensabschnitt, nämlich in den Rührkesselbioreaktor 4.

Das erfindungsgemäße Verfahren wird nachstehend an Hand von Ausführungsbeispielen näher erläutert.

Beispiel 1:

Als Nährmedium wird ein Medium folgender Zusammensetzung genommen:

Xylose (wasserfrei) 60g/l
$KH_2PO_4$ 1g/l
$K_2HPO_4$ 1g/l
$(NH_4)_2SO_4$ 2g/l
$MgSO_4.7H_2O$ 0,1g/l
NaCl 0,01g/l
$Na_2MoOO_4.2H_2O$ 0,01g/l
$CaCl_2$ 0,01g/l
$MnSO_4.H_2O$ 0.015g/l
$FeSO_4.7H_2O$ 0.015g/l
Biotin 0,1mg/l
Thiaminiumchlorid 0,002g/l
p-Aminobenzoesäure 0,002g/l
$Na_2S_2O_4$ 0,035g/l
Resazurin 0,001g/l
Hefeextrakt 0,5g/l
$Na_4OOCCH_3$ 2.2g/l.

Als Gärungsorganismus wird Clostridium acetobutylicum ATCC 824 eingesetzt. Der Gärungsorganismus wird in entsprechender Vorkultur herangezogen, bevor er in das erfindungsgemäße Verfahren eingeführt wird.

Das Nährmedium wird aus dem Vorratsbehälter 1 kommend in den Rührkesselbioreaktor 4 eingeleitet, u.zw. mit einer Verdünnungsrate von 0,3 x $h^{-1}$. Das Inokulum beträgt dabei etwa 10 % des Inhalts des Rührkesselbioreaktors 4. Die Fermentation wird im Rührkesselbioreaktor bei einer Temperatur von 37 °C geführt, wobei innerhalb des Reaktors ein pH von 5,1 gehalten wird. Durch die gewählte Verdünnungsrate wird die Zelldichte innerhalb des Bioreaktors 4 etwa gleich gehalten, wobei die auf Grund des Bakterienwachstums entstehenden neuen Zellen über die Leitung 5 in den Festbettreaktor 7 eingebracht werden, wo sie durch Adhäsion an dem Trägermaterial haften bleiben. Als Trägermaterial wird offenporiges Glassintermaterial verwendet, wobei das Volumen des Glassintermaterials 50 % des Gesamtarbeitsvolumens beträgt. Im Festbettreaktor 7 wird eine Fermentationstemperatur von 33 °C eingehalten, wobei die Fermentation bei einem pH von 4,3 ausgeführt wird. Die Gesamtverdünnungsrate nach beiden Verfahrensabschnitten beträgt 0,15 x $h^{-1}$. Innerhalb des Festbettreaktors 7 wird der Reaktorinhalt mittels Stickstoff begast, wodurch zufolge von Leiteinrichtungen, welche im Festbettreaktor angeordnet sind, eine Zirkulation erzielt wird.

Die Produktivität und die Ausbeute werden nachstehend in Tabelle 1 zusammengefaßt.

Tabelle 1

| | Rest-Xylose | Zucker-Abbau | Zucker-nutzung | Butanol | Gesaat-LM | LM-Ausbeute | LM-Produktivität | Gesaat Säuren | Säuren-Ausbeute | Opt. Dichte | pH-Wert | Eh-Wert |
|---|---|---|---|---|---|---|---|---|---|---|---|---|
| | [g/l] | [%] | [g/l.h] | [g/l] | [g/l] | [%] | [g/lh] | [g/l] | [%] | | | [mV] |
| D = 0.15 h-1 | 28.59 | 50.98 | 4.46 | 5.7 | 9.30 | 31.28 | 1.395 | 3.06 | 5.88 | 2.765 | 4.32 | -436 |

LM = Lösungsmittel

Eh = Potentialmessung

Fortsetzung auf Seite 10 der bisherigen Beschreibung !

Beispiel 2:

Es wird die gleiche Nährstoffzusammensetzung und das gleiche Inokulum und auch die gleichen Verfahrensbedingungen innerhalb des Rührkesselbioreaktors und des Festbettreaktors gewählt. Am Festbettreaktor wird eine externe Schlaufe angeschlossen, in welcher die gebildeten Produkte kontinuierlich oder chargenweise abgezogen werden. Dazu wird die vom Festbettreaktor abgezogene, mit Produkt angereicherte Flüssigkeit auf eine Temperatur von etwa 40° C aufgeheizt und einer Strippkolonne zugeführt, in welcher der Flüssigkeit Stickstoff als Strippgas im Gegenstrom geführt wird. Das Verhältnis der Volumenströme von Flüssigkeit und Gas beträgt 0,35:1. Das von Produkten weitgehend befreite flüssige Medium wird nach Abkühlung auf Gärtemperatur zum Teil wieder in den Festbettreaktor 7 übergeführt, wobei ein geringerer Teil auch in den Rührkesselbioreaktor 4 rückgeführt werden kann.

Die überschüssige, vom Produkt weitgehend befreite Flüssigkeit wird aus dem System abgezogen.Das an Produkt angereicherte, von der Strippkolonne abgezogene Gas wird über eine Kühleinrichtung geführt, in welcher die Produkte kondensieren und abgezogen werden können. Die erzielten steady state-Werte sind nachstehend in Tabelle 2 zusammengefaßt.

Tabelle 2

| | Rest - Xylose [g/l] | Zuk= ker- Ab= bau [%] | Zuk= ker- nut= zung [g/l.h] | Bu- ta- nol [g/l] | Ge= saat- LM [g/l] | LM- Aus- beu= te [%] | LM- Pro= duk= tivi- tät [g/lh] | Ge= saat Säu= ren [g/l] | Säuren- Aus= beute [%] | Opt. Dich= te | pH- Wert | Eh- Wert [mV] |
|---|---|---|---|---|---|---|---|---|---|---|---|---|
| D=0.15 h-1 | 21.50 | 62.50 | 5.37 | 6.82 | 10.87 | 30.36 | 1.638 | 3.76 | 6.59 | 2.41 | 4.10 | -450 |

LM = Lösungsmittel
Eh = Potentialmessung

Wie aus den Tabellen ersichtlich, ist die Produktivität und der Zuckerabbau bei dem Verfahren gemäß Beispiel 2, also bei kontinuierlicher Produktentnahme in einer externen Schlaufe deutlich besser. Dieses Ergebnis zeigt sich auch dann, wenn, wie die vorstehenden Versuche zeigten, die Gärung über einen längeren Zeitraum, vorstehend über 500 Stunden, geführt wurde.

## Patentansprüche

1. Verfahren zur Vergärung von kohlenhydrathältigen Medien mit Hilfe von Butanol, Aceton, Ethanol und/oder Isopropanol bildenden Bakterien, welches Verfahren wenigstens zweistufig ausgeführt wird, wobei im ersten Verfahrensabschnitt im wesentlichen die Bakterien kontinuierlich kultiviert und im zweiten, kontinuierlich oder chargenweise geführten Verfahrensabschnitt im wesentlichen das Produkt gebildet wird, dadurch gekennzeichnet, daß die Bakterien im zweiten Verfahrensschritt adhäsiv bzw. adsorptiv an offenporiges Sinterglas, Bimsstein oder ein gleichwertiges Trägermaterial immobilisiert werden, welches einen hohen $SiO_2$-Gehalt, eine offenporige Struktur, eine Porengröße von 20 - 200 $\mu$m und eine Wasseraufnahmefähigkeit von wenigstens 0,35 ml/cm$^3$ aufweist, und wobei das Verhältnis des Volumens des Trägermaterials zum Gesamtarbeitsvolumen 1:10 bis 1:1,1, beträgt.

2. Verfahren nach Anspruch 1, dadurch gekennzeichnet, daß die Porengröße 50 - 100 $\mu$m beträgt und das Verhältnis des Trägermaterials zum Gesamtarbeitsvolumen 1:5 bis 1:1,6 beträgt.

3. Verfahren nach Anspruch 1 oder 2, dadurch gekennzeichnet, daß aus der Produktbildungsstufe Medium abgezogen, das gebildete Produkt extraktiv, vorzugsweise in an sich bekannter Weise mittels höherer Alkohole oder höherer Fettsäuren, abgetrennt und die dadurch anfallende produktarme Phase in die Produktbildungsstufe und/oder den ersten Verfahrensabschnitt rückgeführt wird.

4. Verfahren nach Anspruch 1 oder 2, dadurch gekennzeichnet, daß im zweiten Abschnitt Produkt, vorzugsweise kontinuierlich, pervaporativ aus dem Medium abgeführt wird.

5. Verfahren nach Anspruch 4, dadurch gekennzeichnet, daß für die pervaporative Produktentnahme eine Pervaporationsmembran, insbesondere aus Silikonkautschuk, eingesetzt wird, wobei eine Temperatur von 30°-90°C, vorzugsweise 40°-80°C, eingehalten wird, und wobei die Dampfdruckerniedrigung pervaporationsseitig durch ein Trägergas, wie $N_2$, $H_2$, $CO_2$ oder eine Mischung dieser Gase, oder durch Fermentationsgas bzw. durch Anlegen eines Vakuums erzielt wird.

**6.** Verfahren nach Anspruch 1 oder 2, dadurch gekennzeichnet, daß im zweiten Abschnitt eine, vorzugsweise kontinuierliche, Produktabtrennung mittels Gasstrippen durchgeführt wird.

**7.** Verfahren nach Anspruch 6, dadurch gekennzeichnet, daß für die kontinuierliche Produktabtrennung durch Gasstrippen ein Strippgas, wie $N_2$, $H_2$, $CO_2$ oder Mischungen dieser Gase, bzw. Fermentationsgas eingesetzt wird, wobei das Verhältnis der Volumensströme von Flüssigkeit zu Gas 0,05:1 - 1,6:1 und die Temperatur 30°-90°C, vorzugsweise 40°-80°C, beträgt.

**8.** Verfahren nach einem der Ansprüche 1 bis 7, dadurch gekennzeichnet, daß der Zulauf an Nährlösung und/oder Zuckerlösung in Abhängigkeit vom Produktgehalt des zweiten Verfahrensabschnittes geregelt wird.

**9.** Vorrichtung zur Durchführung des Verfahrens nach einem der Ansprüche 1 bis 8, dadurch gekennzeichnet, daß ein Bioreaktor und ein mit Bakterien rückhaltendem Material bestückter Reaktor hintereinandergeschaltet sind, wobei an letzterem Reaktor eine externe Schlaufe zur Produktabtrennung angeschlossen ist.

**10.** Vorrichtung nach Anspruch 9, dadurch gekennzeichnet, daß der mit Bakterien rückhaltendem Material bestückte Reaktor ein Festbettreaktor (7) ist, der mit ein oder mehreren Leiteinrichtungen für Gas bzw. Leitungen zum gerichteten Einströmen von vorzugsweise rückgeführtem Medium versehen ist.

**11.** Vorrichtung nach Anspruch 9 oder 10, dadurch gekennzeichnet, daß in der externen Schlaufe zur kontinuierlichen Produktabtrennung eine Gegenstromstrippkolonne (44) oder ein Pervaporationsmodul (13) angeordnet ist, in dem eine Membran (14), insbesondere eine Silikonkautschukmembran, vorgesehen ist.

**Claims**

**1.** A process for the fermentation of media containing carbohydrates, by using bacteria which produce butanol, acetone, ethanol and/or isopropanol, which process comprises at least two stages, wherein the bacteria are substantially continuously cultivated in the first process stage, and in the second, continuous or batchwise run process stage substantially the product is formed, characterised in that in the second process stage the bacteria are immobilized by adhesion or adsorption, respectively, directly on open-porous sinterglass, pumice or an equivalent carrier material with a high $SiO_2$-content, having an open-porous structure, a pore size of 20 - 200 $\mu$m and a water-absorbency of at least 0.35 ml/cm$^3$, and wherein the ratio of the carrier material volume to the total working volume amounts to 1:10 to 1:1.1.

**2.** A process according to claim 1, characterised in that the pore size is 50 to 100 $\mu$m and the ratio of carrier material to total working volume amounts to 1:5 to 1:1.6.

**3.** A process according to claim 1 or 2, characterised in that medium is withdrawn from the product-formation stage, the product formed is separated extractively, preferably in a manner known per se, by means of higher alcohols or higher fatty acids, and that the resulting phase with a low product content is returned into the product formation stage and/or the first process stage.

**4.** A process according to claim 1 or 2, characterised in that product is, preferably continuously, removed pervaporatively from the medium in the second stage.

**5.** A process according to claim 4, characterised in that a pervaporation membrane, in particular consisting of silicone rubber, is used for the pervaporative product removal, wherein a temperature of 30°-90°C, preferably 40°-80°C, is maintained, and wherein the vapour pressure reduction on the pervaporation side is achieved using a carrier gas, such as $N_2$, $H_2$, $CO_2$, or a mixture of these gases, or by fermentation gas or by application of vacuum.

**6.** A process according to claim 1 or 2, characterised in that in the second stage a, preferably continuous, product separation is carried out by gas stripping.

7. A process according to claim 6, characterised in that a stripping gas, such as $N_2$, $H_2$, $CO_2$, or mixtures of these gases, or a fermentation gas is used for the continuous product separation by gas stripping, the ratio of the volume flows of liquid to gas being 0.05:1 - 1.6:1, and the temperature being 30°-90°C, preferably 40°-80°C.

8. A process according to any one of claims 1 to 7, characterised in that the input of nutrient and/or sugar solution is regulated depending on the product content of the second process stage.

9. An arrangement for carrying out the process according to any one of claims 1 to 8, characterised in that a bioreactor and a reactor equipped with material which retains bacteria are connected in series, an external loop for product separation being connected to the latter reactor.

10. An arrangement according to claim 9, characterised in that the reactor equipped with a material which retains bacteria is a solid bed reactor (7) provided with one or several duct means for gas or ducts for the directed inflow of preferably recirculated medium.

11. An arrangement according to claim 9 or 10, characterised in that a counter-current stripping column (44) or a pervaporation module (13) is arranged in the external loop for continuous product separation, in which a membrane (14), in particular a silicon rubber membrane, is provided.

## Revendications

1. Procédé de fermentation de milieux contenant des hydrates de carbone à l'aide de bactéries formant du butanol, de l'acétone, de l'éthanol et/ou de l'isopropanol, lequel procédé est effectué au moins en deux étapes, les bactéries étant essentiellement cultivées en continu dans la première section de procédé et le produit étant formé essentiellement dans la deuxième section de procédé, conduite en continu ou en discontinu, caractérisé en ce que les bactéries sont immobilisées dans la deuxième section de procédé par adhésion, respectivement par adsorption sur du verre fritté à pores ouvertes, sur de la pierre ponce ou sur un matériau vecteur équivalent, qui présente une teneur en $SiO_2$ élevée, une structure à pores ouvertes, une grandeur de pores de 20 - 200 microns et une capacité d'adsorption d'eau d'au moins 0,35 ml/cm$^3$, et le rapport du volume du matériau vecteur au volume de travail total étant de 1:10 à 1:1,1.

2. Procédé selon la revendication 1, caractérisé en ce que la grandeur de pores est de 50 - 100 microns et en ce que le rapport du matériau vecteur au volume de travail total est de 1:5 à 1:1,6.

3. Procédé selon la revendication 1 ou 2, caractérisé en ce que l'on retire le milieu de l'étape de formation de produit, en ce que l'on sépare le produit formé de manière extractive, de préférence d'une manière connue en soi à l'aide d'alcools supérieurs ou d'acides gras supérieurs, et en ce que l'on reconduit la phase pauvre en produit qui en résulte dans l'étape de formation de produit et/ou dans la première section de procédé.

4. Procédé selon la revendication 1 ou 2, caractérisé en ce que, dans la deuxième section, l'on évacue du milieu le produit, de préférence de manière continue, par pervaporation.

5. Procédé selon la revendication 4, caractérisé en ce que l'on utilise, pour le prélèvement de produit par pervaporation, une membrane de pervaporation, en particulier en caoutchouc aux silicones, une température de 30° - 90°C, de préférence de 40° - 80°C étant maintenue, et l'abaissement de pression de vapeur étant obtenue du côté pervaporation par l'intermédiaire d'un gaz vecteur, comme $N_2$, $H_2$, $CO_2$ ou d'un mélange de ces gaz, par l'intermédiaire d'un gaz de fermentation, respectivement par l'application d'un vide.

6. Procédé selon la revendication 1 ou 2, caractérisé en ce que, dans la deuxième section, on effectue une séparation de produit, de préférence en continu, grâce à un strippage au gaz.

7. Procédé selon la revendication 6, caractérisé en ce que, en vue de la séparation continue de produit grâce au strippage au gaz, on utilise un gaz de strippage comme $N_2$, $H_2$, $CO_2$ ou un mélange de ces gaz, respectivement un gaz de fermentation, le rapport des courants volumiques de liquide au gaz étant de 0,05:1 - 1,6:1, et la température étant de 30° - 90°C, de préférence de 40° - 80°C.

8. Procédé selon l'une quelconque des revendications 1 à 7, caractérisé en ce que l'apport en solution nutritive et/ou en solution de sucre est réglée en fonction de la teneur en produit de la deuxième section de procédé.

9. Dispositif de mise en oeuvre du procédé selon l'une quelconque des revendications 1 à 8, caractérisé en ce que qu'un bioréacteur et qu'un réacteur équipé d'un matériau retenant les bactéries sont placés l'un à la suite de l'autre, une boucle externe destinée à la séparation de produit étant raccordée au dernier réacteur.

10. Dispositif selon la revendication 9, caractérisé en ce que le réacteur équipé d'un matériau retenant les bactéries est un réacteur à lit fixe (7), qui est pourvu d'un ou de plusieurs dispositifs directeurs pour le gaz, respectivement de conduites destinées à l'admission dirigée du milieu reconduit de préférence.

11. Dispositif selon la revendication 9 ou 10, caractérisé en ce que, dans la boucle externe destinée à la séparation continue de produit est disposée une colonne de strippage à contre-courant (44) ou un module de pervaporation (13), dans lequel est prévue une membrane (14), en particulier une membrane en caoutchouc aux silicones.

PERVAPORATION
HEIZUNG
GAS
EFFL.
KÜHLUNG
PRODUKTE
Fig. 1

EXTRAKTION
HEIZUNG
MISCHER
EXTRAKTIONSMITTEL
ABSCHEIDER
EXTRAKT.M.+
PRODUKTE
EFFL.
Fig. 2

Fig. 3
STRIPPEN
HEIZUNG
STRIPPKOLONNE
KÜHLUNG
PRODUKTE
GAS
EFFL.